# EUROPEAN PATENT APPLICATION

(11) **EP 4 635 479 A1**
(43) Date of publication of application: **22.10.2025**
(21) Application number: 22968181.2
(22) Date of filing: 15.12.2022
(51) Int. Cl.: A61K 9/08, A61K 47/14, A61K 31/05, A61K 47/06, A61K 8/37

(54) **HYDROXYDIPHENYLETHANE COMPOUND OINTMENT, USE THEREOF, AND METHOD FOR PREPARING SAME**

(71) Applicant: Cutia Therapeutics (Wuxi) Co., Ltd., Wuxi, Jiangsu 214028 (CN)
(72) Inventor: LEI, Lei, Wuxi, Jiangsu 214028 (CN); ZHAO, Liyang, Wuxi, Jiangsu 214028 (CN); CHEN, Yixin, Wuxi, Jiangsu 214028 (CN); ZHANG, Lindong, Wuxi, Jiangsu 214028 (CN); ZHOU, Yingcong, Wuxi, Jiangsu 214028 (CN); LI, Changhui, Wuxi, Jiangsu 214028 (CN); LV, Boyang, Wuxi, Jiangsu 214028 (CN); JIN, Yinghua, Wuxi, Jiangsu 214028 (CN); WANG, Dan, Wuxi, Jiangsu 214028 (CN)
(74) Representative: Barker Brettell LLP
(86) International application number: PCT/CN2022/139225
(87) International publication number: WO 2024/124471

(57) **Abstract**

Provided are a solution-like hydroxydiphenylethane compound ointment, use thereof, and a method for preparing same. The ointment comprises: 0.001 wt%-3 wt% of an active ingredient hydroxydiphenylethane compound, 0.01%-1% of an antioxidant, and the balance of an ointment matrix. The ointment matrix comprises vaseline and/or liquid paraffin, a medium-chain triglyceride, and isopropyl palmitate and/or isopropyl myristate. The hydroxydiphenylethane compound ointment features good drug solubility and transdermal performance, good photostability, and no need for shading in use, and is thus suitable for use on areas exposed to the light, such as the head, face, and other regions.

## Description

### TECHNICAL FIELD

The invention relates to an external-use composition of hydroxydiphenylethane compounds, in particular to a solution-type ointment preparation suitable for poorly soluble and light-unstable hydroxydiphenylethane compounds, and its use and preparation method.

### BACKGROUND OF THE INVENTION

Ointment refers to a uniform semi-solid topical preparation made by mixing active pharmaceutical ingredient(API) with oily or water-soluble matrix. It is one of the most widely used topical preparations. Ointment can form a closed barrier on the skin surface with good moisturizing effect. It is particularly suitable for some skin diseases, such as atopic dermatitis with damaged skin barrier. According to the different dispersion states of API in the matrix, ointment can be divided into solution-type ointment and suspension-type ointment.

The most widely used ointment matrix is Vaseline. Due to the limited solubility of drugs in Vaseline, most commercially available ointments are suspension-type ointments. However, in suspension-type ointments, the API is evenly dispersed in the matrix in the form of fine powder, only a very small amount of API dissolved in the matrix in a molecular state can penetrate into the skin, while most of the suspended API particles cannot diffuse into the skin. Therefore, it is impossible to achieve a higher intradermal drug concentration, the drug takes effect more slowly, and the drug that fails to enter the skin is wasted more. Different batches of API powders have slight differences in particle size distribution, water content, particle morphology, etc., which may lead to batch differences in ointments and affect their clinical effectiveness and safety. In addition, drugs with polymorphic forms may also undergo crystal transformation during production and storage, which not only poses greater challenges to the process, but also likely to affect the quality of the product.

In solution-type ointments, the API is dissolved or co-dissolved in the matrix or matrix components. Dissolution process of changing API from powder to molecular form for the purpose of transdermal absorption is not necessary. The API in molecular form can diffuse out of the matrix better and penetrate into the skin. The batch-to-batch variability of solution-type ointments is relatively small, which is more conducive to the quality control and clinical use of ointments.

Although solution-type ointments have many technical advantages, many commonly used solubilizers and penetration enhancers such as propylene glycol, polyethylene glycol, diethylene glycol monomethyl ether, ethanol, etc. are not miscible with the ointment matrix. In addition, the amount of solubilizer is crucial to the preparation of solution-type ointments. Insufficient amount of solubilizer can lead to the drug being near its saturation solubility, which may cause crystallization and precipitation of the drug due to changes in external conditions or long-term storage. Excessive amount of solubilizer can lead to an overly high solubility of the drug in the formulation, which may cause the drug to remain within the formulation and hinder its partitioning into the skin. Therefore, there are large technical challenges in selecting suitable excipients to prepare homogeneous solution-type ointments.

A variety of substitution reactions can occur on the two benzene rings of diphenylethane compounds, and various pharmaceutical activities such as anti-tumor, anticoagulant, and anti-inflammatory can be produced depending on the substituents. There are few studies on diphenylethane compounds in the field of skin diseases and beauty. KR20150004221A discloses an androgen receptor antagonist with a diphenylethane structure, which may be used in related diseases such as hair loss and acne, but the patent does not involve its topical preparations. CN108366938B discloses a biaromatic vitamin D receptor agonist with a diphenylethane structure, and provides a cosmetic composition that may be used for anti-wrinkle, the above composition is quite different from the ointment prescription of the present invention, and the active ingredient has no phenolic hydroxyl substitution, so there is no need to solve the problem of compound instability. CN103483158B discloses a class of diphenylethane compounds that can be used to treat immune diseases or inflammation, it also provides an external cream composition that shows good effects of inhibiting edema and reducing inflammation in the TPA-induced mouse skin edema model. However, such compounds have extremely poor solubility, and the compositions disclosed in the patent use high concentrations of propylene glycol and ionic surfactants, which are irritating to the skin.

On the other hand, diphenylethane derivatives containing hydroxyl groups are very easy to be degraded by light, so the color and performance of products comprising the composition change under light. This light instability limits its application in skin diseases and cosmetics. Usually, light protectants can be added to the preparation to improve the light stability of the active pharmaceutical molecule. Light protectants can be divided into UV blockers and UV absorbers from the mechanism. The former, such as titanium dioxide and zinc oxide, have good protection but usually require a large dosage, and the solid powder affects the skin feel of the preparation, the latter, such as octyl salicylate, has a certain degree of transdermal absorption and is often allergenic or acne-causing. These light protectants are widely used in sunscreen cosmetics, but there are fewer light protectants suitable for pharmaceutical preparations, and the dosage is limited.

### SUMMARY OF THE INVENTION

### Technical problems to be solved by the invention

Most commercially available ointments for poorly soluble drugs are suspension-type ointments, and there are still many technical challenges in developing homogeneous, stable solution-type ointments. How to select appropriate excipients and optimize their dosage, so that they can dissolve the drug without crystallization, penetrate the skin well in molecular form, and not affect the transdermal absorption of the drug due to reduced thermodynamic activity, is still a process of continuous trial and error and accumulation of experience.

Many drugs have multiple clinical specifications for different populations or different disease conditions. In order to achieve the best clinical effect, the formulation prescription and/or process need to be adjusted for different specifications. Solution-type ointments need to optimize the dosage of solubilizers and penetration enhancers for different specifications to achieve optimal transdermal drug absorption. This often requires a lot of resources and time, and it is difficult to directly test the solubility of drugs in formulations, and there is a lack of systematic and scientific methods to guide formulation optimization.

In view of the above technical problems, the purpose of the present invention is to provide a solution-type ointment suitable for hydroxydiphenylethane compounds, screen suitable solubilizers and penetration enhancers, and establish a mathematical model that can predict the solubility of drugs in multi-component systems. The optimal prescription ratio can be selected according to the dosage of active ingredients to prepare a uniform and stable external preparation with good drug release and transdermal performance.

In addition, hydroxydiphenylethane compounds are unstable to light and heat, and are easily oxidized and degraded under light. The colored quinone degradation products may also cause skin pigmentation. Therefore, they need to be shielded from light during use, and cannot be used in places exposed to light such as the head and face. However, the head and face are prone to many skin diseases, which limits the clinical application range of hydroxydiphenylethane active ingredients and affects patient compliance. Another purpose of the present invention is to provide a hydroxydiphenylethane compound topical composition that has good light stability, does not require light shielding during use, and can be applied to the head and face.

### Technical solutions

In order to solve the above technical problems, the present invention provides a solution-type ointment of hydroxydiphenylethane compounds, which can provide the best auxiliary material combination and ratio according to the concentration of the active ingredient, so that the active ingredient can be fully dissolved in the ointment matrix, and provide good drug release and transdermal absorption, and can be used for affected areas exposed to light such as the head and face.

The hydroxydiphenylethane compound solution-type ointment provided by the present invention comprises 0.001% to 3% by weight of an active ingredient hydroxydiphenylethane compound and the balance of an ointment matrix, the ointment matrix comprises vaseline and/or liquid paraffin, medium-chain triglyceride, and isopropyl palmitate and/or isopropyl myristate.

The inventors have found through stud that in the solution-type ointment system of the present invention, the active ingredient hydroxydiphenylethane compound exhibit anti-inflammatory, soothing and other pharmacological effects within a concentration range of 0.001% to 3% by weight, and are feasible to be drug.

Wherein, the hydroxydiphenylethane compound is selected from 1,2-bis(3,5-dihydroxy-4-isopropylphenyl)ethane, 2-isopropyl-5-(2'-phenylethyl)-1,3-benzenediol, 1-(3,5-dihydroxy-4-ethylphenyl)-2-phenylethane, 1,2-bis(3,5-dihydroxy-4-ethylphenyl)ethane, 1-(3,5-dihydroxy-4-butylphenyl)-2-phenylethane, 1,2-bis(3,5-dihydroxy-4-butylphenyl)ethane, preferably 2-isopropyl-5-(2'-phenylethyl)-1,3-benzenediol.

The solution-type ointment of the present invention further contains an antioxidant, which is an oil-soluble antioxidant selected from one or more of propyl gallate, butylated hydroxyanisole, and dibutylhydroxytoluene, preferably propyl gallate (PG).

In the solution-type ointment of the present invention, the total amount of medium-chain triglyceride, isopropyl palmitate and isopropyl myristate is 2-30% by weight, preferably 5-25% by weight. When the formulation specification is not higher than 1% by weight, the total amount is preferably 5-17% by weight; if the formulation specification is 1-3% by weight, the total amount is preferably 13%-25% by weight.

In the hydroxydiphenylethane compound ointment, the amount of isopropyl palmitate and/or isopropyl myristate is 0.5-15% by weight, preferably 2-15% by weight, and more preferably 4-12% by weight.

In the solution-type ointment of the present invention, the ratio of medium-chain triglycerides to isopropyl palmitate and/or isopropyl myristate is 1:4-3:1, preferably 1:2-2:1, and more preferably 1:1-2:1.

In the solution-type ointment of the present invention, the saturated solubility of the active ingredient in the composition can be predicted by the following mathematical model:
Saturated solubility (%) = 0.445829*A + 0.032300*B - 0.000599*C + 0.008112*A*B - 0.003841*A*C. Wherein A, B, and C represent the content of medium-chain triglyceride (%), the content of isopropyl myristate and/or isopropyl palmitate (%), and the content of hydrocarbon compounds (%), respectively, and A+B+C = 100. Therefore, when the content of the active ingredient does not exceed 0.445829×A+0.032300×B-0.000599×C+0.008112×A×B-0.003841×A×C, a better solution-type ointment can be prepared. In practical applications, it is also acceptable that the content of the active ingredient does not exceed 1.2 times the saturated solubility. From the perspective of minimizing the risk of precipitation, it is preferred that the content of the active ingredient is no higher than 90%×saturated solubility.

In the solution-type ointment of the present invention, the content of the hydroxydiphenylethane compound is 0.005% to 2% by weight, the content of the antioxidant is 0.01% to 1% by weight, more preferably the content of the hydroxydiphenylethane compound is 0.005% to 1% by weight, the content of the antioxidant is 0.02% to 0.5% by weight, and the content of the ointment matrix is 97.5% to 99.97% by weight.

In the solution-type ointment of the present invention, the active ingredient is present in the ointment in a non-crystalline form.

The solution-type ointment of the present invention contains substantially no water or contains less than 2% by weight of water.

The hydroxydiphenylethane compound ointment of the present invention can be used on the skin of the whole body, especially on the head and/or face.

The present invention also provides a method for preparing a hydroxydiphenylethane compound ointment, wherein, the active ingredient hydroxydiphenylethane compound (together with the antioxidant when containing an antioxidant) is added to medium-chain triglycerides, isopropyl myristate and/or isopropyl myristate, heated and stirred to dissolve, and then added to melted vaseline and/or liquid paraffin, stirred evenly and cooled to room temperature to obtain the ointment.

### Beneficial technical effects

The present invention discloses a solution-type ointment of hydroxydiphenylethane compounds. The composition creatively uses a compounding of medium-chain triglycerides and isopropyl palmitate and/or isopropyl myristate. Medium-chain triglycerides not only have a good solubilizing effect on 2-isopropyl-5-(2'-phenylethyl)-1,3-benzenediol with extremely poor solubility, but also stabilize the entire ointment system to prepare a homogeneous and stable solution-type ointment. Isopropyl myristate can not only interact with stratum corneum lipids to promote transdermal absorption of drug, it can also form a reservoir in the skin to continuously and slowly release drug.

In the prior art, medium-chain triglycerides and isopropyl palmitate/isopropyl myristate are only used as the oil phase of the emulsion, or as a common transdermal penetration enhancer. Different from the prior art, the present invention unexpectedly finds that the compounding of these two excipients with hydroxydiphenylethane compounds can form a homogeneous ointment in a hydrocarbon ointment matrix, and the active pharmaceutical ingredient (hereinafter sometimes referred to as API) exists in a non-crystalline dissolved form in the ointment. Through study, the inventors found the ratio principle of hydroxydiphenylethane compounds in the compounding system in a dissolved form, that is, the mathematical relationship between the amount of hydroxydiphenylethane compounds and the amount of medium-chain triglyceride, isopropyl palmitate/isopropyl myristate, and hydrocarbon compounds. Only when this mathematical relationship is met can a solution-type ointment be formed.

According to the preferred dosage and ratio of the present invention, the active ingredient in the composition can be dissolved in the ointment matrix in molecular form, even after being stored at 5°C for 3 months, 2-isopropyl-5-(2'-phenylethyl)-1,3-benzenediol with a high concentration will not crystallize. On the other hand, the composition maintains high thermodynamic properties and drug release rate. Good transdermal absorption is still maintained at low drug concentrations, and the drug can be effectively released from the ointment matrix and can pass through the stratum corneum barrier to reach the required therapeutic concentration at the site of action.

In addition, the present invention also solves the problem of light instability of hydroxydiphenylethane compounds. The antioxidant used in the composition effectively protects the active ingredient, so that this type of compound can be used on parts of the body directly exposed to light, such as the head and/or face, and there is no need to deliberately avoid light during use, which expands the scope of use of this type of compounds and improves patient compliance.

### BRIEF DESCRIPTION OF THE DRAWINGS

FIG.1 is a contour plot showing the solubility prediction equation for 2-isopropyl-5-(2'-phenylethyl)-1,3-benzenediol in a visual representation form.
FIG.2 is a three-dimensional graph showing the solubility prediction equation for 2-isopropyl-5-(2'-phenylethyl)-1,3-benzenediol in a visual representation form.
FIG. 3 shows the results of the light stability test of the cream preparation of Comparative Example 1.
FIG. 4 shows the results of a light stability test of the ointment of Example 10 of the present invention.
FIG. 5 shows a polarizing microscope photograph (a) of the solution-type ointment of Example 15 of the present invention and a polarizing microscope photograph (b) of the non-solution-type ointment of Comparative Example 4.
FIG. 6 shows the in vivo exposure of topical administration of the ointment of Example 16 (solid line) and Example 17 (dashed line) in the present invention.

### DETAILED DESCRIPTION OF THE EMBODIMENTS

In the present invention, the compound 2-isopropyl-5-(2'-phenylethyl)-1,3-benzenediol, sometimes also referred to as 1-(3,5-dihydroxy-4-isopropylphenyl)-2-phenylethane, is a compound having the following structural formula.

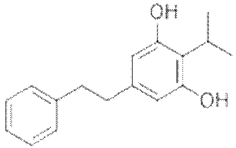

### EXAMPLE

The present invention is described in detail below with reference to the following examples. The examples provide detailed implementation methods and processes to fully disclose and demonstrate that the invention is implementable. The examples do not represent limitations on the invention, and the protection scope of the present invention is not limited to the following examples.

The experimental methods in the embodiments of the description that do not specify specific conditions are usually carried out according to conventional conditions in the art or according to conditions recommended by the manufacturer. In the present invention, unless otherwise specified, "parts" means parts by weight, and "%" means percentage by weight. In the present invention, unless otherwise specified, "or above", "or below", "within" means including the number itself.

In the present invention, white vaseline, liquid paraffin, medium-chain triglycerides, isopropyl palmitate, isopropyl myristate, propylene glycol, cetyl alcohol, Glyceryl Monostearate and Diglycerides(also be simply referred to as Glyceryl Stearate), Tween 80, purified water, dibutylhydroxytoluene (BHT), butylated hydroxyanisole (BHA), vitamin E (VE), propyl gallate (PG), 2-ethylhexyl salicylate(also referred as Octyl salicylate) (OSAL), etc., are commercially available products.

### Preparation of Hydroxydiphenylethane Compound Ointment

The preparation method is as follows: heat and melt a hydrocarbon compounds (such as vaseline or a mixture of vaseline and liquid paraffin), stir until uniform and keep warm for later use. Weigh the required amount of medium-chain triglycerides, isopropyl palmitate and/or isopropyl myristate in another suitable container, stir and heat to the required temperature, then add the active ingredient hydroxydiphenylethane compound (if an antioxidant is used in the ointment, add the antioxidant at the same time), stir to dissolve, then pour into the above melted vaseline and liquid paraffin, stir and cool to room temperature, and the ointment is obtained.

### Example 1 Screening of solubilizers and penetration enhancers

The inventor determined saturated solubility of 2-isopropyl-5-(2'-phenylethyl)-1,3-benzenediol in different excipients, and found that Span 80 (solubility 3%), 10% glycerol (solubility = 0.00007%), water (solubility = 0.0008%), 20% ethanol (solubility = 0.02%), and light liquid paraffin (solubility = 0.01%) could not dissolve an effective dose of 2-isopropyl-5-(2'-phenylethyl)-1,3-benzenediol, and therefore a solution-type ointment could not be prepared. The inventor unexpectedly found that excipients able to provide good solubility are medium-chain triglycerides (solubility = 27%), isopropyl palmitate (solubility = 23%), isopropyl myristate (solubility = 27%), polyethylene glycol 400 (solubility = 30%), diethylene glycol monoethyl ether (solubility = 52%), propylene glycol (solubility = 30%), propylene glycol monolaurate (solubility = 30%), and polysorbate 80 (solubility = 17%).

The above-mentioned excipients that can provide good solubility were further used in combination with hydrocarbon compounds (light liquid paraffin) and 1% 2-isopropyl-5-(2'-phenylethyl)-1,3-benzenediol to investigate the physical stability of the resulting ointment. Liquid paraffin has similar properties to vaseline, so liquid paraffin was used instead of solid vaseline to carry out the prescription screening experiment. All components were stirred evenly at room temperature, and the test sample was obtained after high-speed homogenization. After standing overnight, observe whether the sample is stratified. The results showed that common API solvents, such as propylene glycol, polyethylene glycol 400, diethylene glycol monoethyl ether, polysorbate 80, propylene glycol monolaurate, etc., did not have good compatibility with API in the hydrocarbon compound matrix, and stratification occurred, and a solution-type ointment could not be formed. The specific formulation and results are shown in Table 1:

**Table 1. Screening of solubilizers and penetration enhancers**

| Formulati on | 1 | 2 | 3 | 4 | 5 | 6 | 7 | 8 | 9 |
|---|---|---|---|---|---|---|---|---|---|
| 2-isopropyl-5-(2'-phenyleth yl)-1,3-benzenedi ol | 1% | 1% | 1% | 1% | 1% | 1% | 1% | 1% | 1% |
| light liquid paraffin | 91.5% | 91.5% | 91.5% | 71% | 91.5% | 67.7% | 72% | 71% | 71.7% |
| diethylene glycol monoethyl ether | 5% | 5% | 5% | 4% | - | - | - | - | - |
| Polyglycer ol fatty acid esters | 2.5% | - | - | 4% | - | 4.0% | - | 2% | - |
| Propylene glycol monolaura te | - | 2.5% | - | - | 2.5% | | - | - | - |
| isopropyl palmitate | - | - | 2.5% | - | - | | - | - | 7.3% |
| medium-chain triglycerid e | - | - | - | 20% | - | 20% | 20% | 20% | 20% |
| propylene glycol | - | - | - | - | 5% | 7.3% | - | - | - |
| Polyethyle ne Glycol 400 | - | - | - | - | - | - | 5.2% | 5.2% | - |
| Polyethyle ne Glycol Octadecyl Ether | - | - | - | - | - | - | 1% | - | - |
| Polyethyle ne Glycol Glyceryl Caprylate Decanoate | - | - | - | - | - | - | - | 2% | - |

| Results | | | | | | | | | |
|---|---|---|---|---|---|---|---|---|---|
| Formulati on stability | stratifi ed | stratifi ed | stratifi ed | stratifi ed | stratifi ed | stratifi ed | stratifi ed | stratifi ed | Not stratifi ed |

It was unexpectedly found that the ointment system composed of isopropyl palmitate and medium-chain triglycerides (Formulation 9 in Table 1) was stable and did not stratify. In this system, even if the content of the API was increased to 5% (correspondingly, the content of liquid paraffin in Formulation 9 in Table 1 was reduced by 4%), the prepared ointment system was still stable for a long time, and the microstructure of the ointment was observed using a polarizing microscope, and no API crystals were observed, indicating that the API existed in the ointment in a non-crystalline molecular state.

### Example 2 Solubility prediction model and optimization of dosage of isopropyl myristate and medium-chain triglycerides

By designing different formulations and conducting a series of experimental studies, a mathematical model that can predict the saturated solubility of 2-isopropyl-5-(2'-phenylethyl)-1,3-benzenediol in multi-component ointments was summarized. The method is as follows:
Experimental variables A, B, and C were set as the content of medium-chain triglycerides (%), isopropyl myristate (%), and light liquid paraffin (%), respectively (for the convenience of experimental design and operation, light liquid paraffin was used to represent all hydrocarbon compounds). According to the maximum safe dosage of excipients (FDA IID database), the variable range was set as follows: 0 < A≤20, 0 < B≤20. According to the preliminary experimental results (medium-chain triglycerides are more important for the dissolution of API and the stability of the system) and the requirements for the viscosity and skin feel of the ointment, the constraints were set as follows: A > B; A+B≤25%; A+B+C = 100%.

According to the variable range and constraint conditions, a total of 20 experimental formulations were designed. After the components were mixed evenly in proportion, an excess of 2-isopropyl-5-(2'-phenylethyl)-1,3-benzenediol was added, stirred overnight at room temperature, centrifuged, the supernatant was filtered and diluted, and the saturated solubility of 2-isopropyl-5-(2'-phenylethyl)-1,3-benzenediol was tested. The component ratios and experimental results of each experimental formulation are shown in Table 2.

**Table 2. DOE test of solubility of API in preparation**

| Experime ntal formulati ons | Content of medium-chain triglycerides (%) (A) | Content of isopropyl myristate (%) (B) | Content of light liquid paraffin (%) (C) | Saturation solubility of 2-isopropyl-5-(2'-phenylethyl)-1,3-benzenediol (%) |
|---|---|---|---|---|
| 1 | 5.97 | 5.97 | 88.06 | 1.09 |
| 2 | 6.63 | 6.63 | 86.74 | 1.22 |
| 3 | 20.00 | 0.00 | 80.00 | 2.47 |
| 4 | 2.00 | 0.00 | 98.00 | 0.11 |
| 5 | 12.50 | 12.50 | 75.00 | 3.78 |
| 6 | 16.15 | 0.00 | 83.85 | 2.07 |
| 7 | 20.00 | 0.00 | 80.00 | 2.91 |
| 8 | 18.79 | 6.21 | 75.00 | 4.13 |
| 9 | 11.30 | 3.90 | 84.80 | 1.73 |
| 10 | 11.62 | 2.02 | 86.36 | 1.55 |
| 11 | 10.84 | 0.00 | 89.16 | 1.05 |
| 12 | 12.50 | 12.50 | 75.00 | 3.42 |
| 13 | 9.35 | 8.77 | 81.88 | 2.20 |
| 14 | 17.87 | 7.13 | 75.00 | 4.11 |
| 15 | 10.84 | 0.00 | 89.16 | 1.06 |
| 16 | 15.17 | 4.03 | 80.80 | 2.65 |
| 17 | 13.29 | 8.31 | 78.40 | 3.98 |
| 18 | 5.84 | 1.16 | 93.00 | 0.48 |
| 19 | 2.00 | 0.00 | 98.00 | 0.09 |
| 20 | 18.79 | 6.21 | 75.00 | 3.95 |

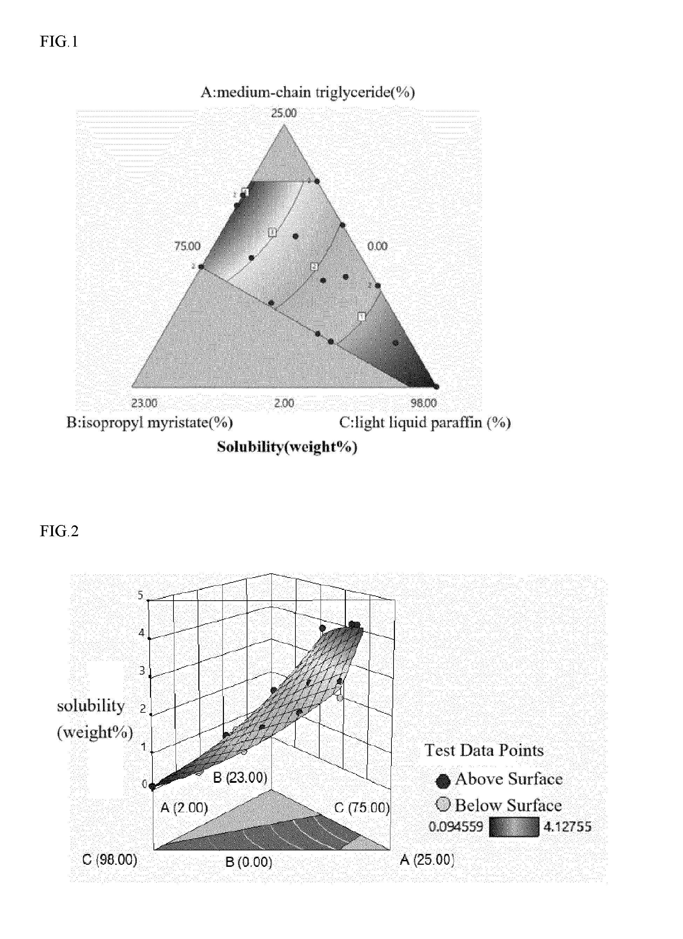

Taking the saturated solubility (%) of 2-isopropyl-5-(2'-phenylethyl)-1,3-benzenediol as the dependent variable and A, B, and C as the independent variables, the data in Table 2 were fitted with a ternary quadratic equation (removing the quadratic term) to obtain the fitting equation of the saturated solubility, that is, saturated solubility (%) = 0.445829 × A + 0.032300 × B - 0.000599 × C + 0.008112 × A × B - 0.003841 × A × C (wherein A, B, and C represent the content of medium-chain triglycerides (%), the content of isopropyl myristate/isopropyl palmitate (%), and the content of white Vaseline/liquid paraffin (%), respectively). The p-value of the fitting equation is less than 0.0001 (the closer the p-value is to 0, the more the equation can represent the principle of the data), and the regression coefficient (R²) value is 0.9932 (the closer the R² value is to 1, the more the equation can represent the principle of the data), and the degree of fit is high. The three-dimensional space surface map of the prediction equation and its two-dimensional space projection map (contour map) are shown in FIG.1 and FIG.2.

The saturated solubility of 2-isopropyl-5-(2'-phenylethyl)-1,3-benzenediol in different proportions of medium-chain triglycerides, isopropyl myristate and light liquid paraffin solution was investigated to verify the accuracy of the prediction equation. The results are shown in Table 3. The equation can accurately predict the solubility of API in different proportions of excipients. In practical applications, it is acceptable that the API content is within the range of no more than 1.2 times the saturated solubility, that is, 120%×(0.445829×A+0.032300×B-0.000599×C+0.008112×A×B-0.003841×A×C). Preferably, when the content of the active ingredient in the preparation is not higher than 0.445829×A+0.032300×B-0.000599×C+0.008112×A×B-0.003841×A×C, a better solution-type ointment can be prepared. It is further preferred that the content of the active ingredient in the preparation is not higher than 90% of the saturated solubility, that is, the content of the active ingredient in the preparation is not higher than 90%×(0.445829×A+0.032300×B-0.000599×C+0.008112×A×B-0.003841×A×C).

**Table 3. Validation of the solubility prediction equation for 2-isopropyl-5-(2'-phenylethyl)-1,3-benzenediol**

| Validation group | Content of medium-chain triglycerides (%) (A) | Content of isopropyl myristate(%) (B) | Content of light liquid paraffin(%) (C) | Measured saturated solubility (%) | Predicted saturated solubility (%) |
|---|---|---|---|---|---|
| 1 | 20 | 0.85 | 79.15 | 3.32 | 2.95 |
| 2 | 20 | 3.4 | 76.6 | 3.85 | 3.65 |
| 3 | 13.75 | 9.23 | 77.02 | 3.52 | 3.34 |
| 4 | 11.2 | 7.55 | 81.25 | 2.56 | 2.38 |
| 5 | 8.72 | 5.04 | 86.24 | 1.58 | 1.47 |
| 6 | 3 | 3 | 94 | 0.401 | 0.368 |
| 7 | 2 | 2 | 96 | 0.203 | 0.194 |

The results of the preliminary experiment showed that if an ointment with satisfactory viscosity and skin feel is to be prepared, the total amount of medium-chain triglycerides and isopropyl myristate should account for less than 30% of the ointment preparation, and preferably, the total amount should be less than 25%. In addition, the saturated solubility of the API increases with the increase in the total amount of medium-chain triglycerides and isopropyl myristate, and the solubilization effect of medium-chain triglycerides is better than that of isopropyl myristate. The preliminary experiment showed that the ratio of medium-chain triglycerides to isopropyl myristate should not be less than 1:4, otherwise the solubility will decrease significantly, and preferably, the amount of medium-chain triglyceride should not be less than isopropyl myristate (not less than 1:1). Therefore, the model constraints are set according to the preliminary experimental results. As shown in Table 3, according to the saturated solubility predicted by the above equation, the concentrations of medium-chain triglycerides and isopropyl myristate required for preparations of different specifications can easily be determined. For example, to prepare a 0.5% concentration of 2-isopropyl-5-(2'-phenylethyl)-1,3-benzenediol solution-type ointment, when A (content of medium-chain triglyceride) is 3% and B (content of isopropyl myristic acid ester) should be at least ≥4.93%, solve the equation 0.5=0.445829×3+0.032300×B-0.000599×(100-3-B)+0.008112×3×B-0.003841×3×(100-3-B), B=4.93%.

### Examples 3-7 In vitro drug release of different formulations

Prepare 2-isopropyl-5-(2'-phenylethyl)-1,3-benzenediol ointment with specification of 1%, containing different proportions of medium-chain triglycerides, isopropyl myristate and liquid paraffin. Using StraM^{®} artificial simulated skin (supplier: Millipore), and 0.5% sodium dodecyl sulfate solution as the receiving medium to investigate the in vitro release rate and cumulative release amount of different formulations within 9 hours. The investigated formulations and their results are shown in Table 4.

**Table 4 Release rate and cumulative drug release amount of ointments with different formulation**

| | Example 3 | Example 4 | Example 5 | Example 6 | Example 7 |
|---|---|---|---|---|---|
| 2-isopropyl-5-(2'-phenylethyl)-1,3-benzenediol(wt%) | 1 | 1 | 1 | 1 | 1 |
| Medium-chain triglycerides(wt%) | 11.5 | 11.88 | 15.47 | 20 | 16 |
| Isopropyl myristate (wt%) | 9.72 | 6.66 | 4.71 | 3.94 | 11.53 |
| Liquid paraffin | 11.28 | 13.96 | 12.32 | 8.57 | 4.98 |
| White vaseline | 66.5 | 66.5 | 66.5 | 66.5 | 66.5 |

| Results | | | | | |
|---|---|---|---|---|---|
| Drug release rate (µg/cm²*h^{1/2}) | 125.95 | 125.59 | 92.77 | 90.55 | 93.9 |
| Cumulative drug release amount (µg) | 488.9 | 480.26 | 348.43 | 340.04 | 360.95 |

The results of the investigation showed that an increase in the dosage of medium-chain triglycerides would slow down the release rate of the drug. However, medium-chain triglycerides play an important role in maintaining the physical stability of the ointment matrix, and their minimum dosage is required to maintain the ointment matrix without precipitation and stratification. Preliminary experimental results showed that when the ratio of medium-chain triglycerides to isopropyl myristate is not less than 1:4, the drug has better solubility in the ointment matrix and does not precipitate in the ointment matrix. The higher the ratio of medium-chain triglycerides to isopropyl myristate, the better the solubilization effect of the formulation, but the drug release rate will also decrease. As shown in Table 4, the total amount of medium-chain triglycerides and isopropyl myristate in Examples 3-6 is similar, when the ratio of the above two ingredients increases from 1.2:1 to 5:1, the drug release rate decreases from 125.95 µg/cm² *h^{1/2} to 90.55 µg/cm² *h^{1/2}, and the cumulative drug release amount decreases from 488.9 µg to 340.04 µg. In particular, when the ratio of medium-chain triglycerides to isopropyl myristate is greater than 3:1, the drug release rate and the cumulative drug release amount begin to decrease significantly. Therefore, the preferred ratio of medium-chain triglycerides to isopropyl myristate is 1:4-3: 1.

Comparing Example 3 with Example 7, the ratio of medium-chain triglycerides to isopropyl myristate is same, and the total amount of the latter 27.53% is higher than the former 21.22%. When the total amount increases from 21.22% to 27.53%, the drug release rate decreases from 125.95 µg/cm² *h^{1/2} to 93.9 µg/cm² *h^{1/2}, and the cumulative drug release amount decreases from 488.90 µg to 360.95 µg. Therefore, when the ratio in the prescription can completely dissolve the API and does not cause the ointment matrix to separate, the total amount of medium-chain triglycerides and isopropyl myristate should be kept as small as possible to obtain the highest possible drug release rate and release amount.

When preparing a 1% ointment, if MCT:IPM=1:1, the amount of MCT and IPM is at least 5.35%; if MCT:IPM=1:4, the amount of MCT is at least 2.6%, and the corresponding amount of IPM is at least 10.4%; if MCT:IPM=3:1, the amount of MCT is at least 7.35%, and the corresponding amount of IPM is at least 2.45%.

More preferably, when preparing a 1% ointment, if MCT:IPM=1:1, the amount of MCT and IPM is at least 6.41%; if MCT:IPM=1:4, the amount of MCT is at least 3.4%, and the corresponding amount of IPM is at least 13.6%; if MCT:IPM=3:1, the amount of MCT is at least 8.85%, and the corresponding amount of IPM is at least 2.95%.

Therefore, when preparing a 1% ointment, a stable solution-type ointment can be prepared when the total amount of medium-chain triglycerides and isopropyl myristate is at least 13%, preferably 17%, and the MCT:IPM ratio is in the range of 1:4-3: 1.

When preparing a 3% cream, if MCT:IPM=1:1, the amount of MCT and IPM is at least 10.55%; if MCT:IPM=1:4, the amount of MCT is at least 5.67%, and the corresponding amount of IPM is at least 22.68%; if MCT:IPM=1:2, the amount of MCT is at least 7.93%, and the corresponding amount of IPM is at least 15.86%; if MCT: IPM=3:1, the amount of MCT is at least 14.67%, and the corresponding amount of IPM is at least 4.89%.

More preferably, when preparing a 3% cream, if MCT:IPM=1:1, the amount of MCT and IPM is at least 11.23%; if MCT:IPM=1:4, the amount of MCT is at least 6.05%, and the corresponding amount of IPM is at least 24.2%; if MCT:IPM=1:2, the amount of MCT is at least 8.32%, and the corresponding amount of IPM is at least 16.64%; if MCT:IPM=3:1, the amount of MCT is at least 15.63%, and the corresponding amount of IPM is at least 5.21%.

Therefore, when preparing a 3% ointment, if the total amount of medium-chain triglycerides and isopropyl myristate is 30%, a stable solution-type ointment can be prepared with a ratio of MCT:IPM in the range of 1:4-3:1. More preferably, if the total amount of medium-chain triglycerides and isopropyl myristate is 25%, a stable solution-type ointment can be prepared with a ratio of MCT:IPM in the range of 1:2-3:1.

### Examples 8-13 Ointments containing different antioxidants

2-isopropyl-5-(2'-phenylethyl)-1,3-benzenediol ointments containing different antioxidants was prepared according to the prescription shown in Table 5.

**Table 5. Ointments containing different antioxidants**

| | Example 8 | Example 9 | Example 10 | Example 11 | Example 12 | Example 13 |
|---|---|---|---|---|---|---|
| 2-isopropyl-5-(2'-phenylethyl)-1,3-benzenediol (wt%) | 0.3 | 0.3 | 0.3 | 0.3 | 0.3 | 0.3 |
| light liquid paraffin (wt%) | 9 | 9 | 9 | 9 | 9 | 9 |
| isopropyl myristate | 11.8 | 11.8 | 11.8 | 11.8 | 11.8 | 11.8 |
| medium-chain triglycerides (wt%) | 11.7 | 11.7 | 11.7 | 11.7 | 11.7 | 11.7 |
| dibutylhydroxytoluene (BHT) (wt%) | 0.5 | N/A | N/A | 0.5 | 0.2 | N/A |
| vitamin E(VE) (wt%) | N/A | 0.5 | N/A | N/A | N/A | N/A |
| propyl gallate (PG) (wt%) | N/A | N/A | 0.1 | 0.05 | 0.1 | N/A |
| white vaseline (wt%) | Qs to 100 | Qs to 100 | Qs to 100 | Qs to 100 | Qs to 100 | Qs to 100 |

### Comparative Example 1

A 3,5-dihydroxy-4-isopropyldiphenylethane comparative cream without an antioxidant was prepared according to the following table:

**Table 6. 2-isopropyl-5-(2'-phenylethyl)-1,3-benzenediol comparative cream (Comparative example 1)**

| Ingredients | Dosage (%,w/w) |
|---|---|
| 2-isopropyl-5-(2'-phenylethyl)-1,3-benzenediol | 0.3 |
| Propylene Glycol | 23.5 |
| White Vaseline | 6.0 |
| Cetyl Alcohol | 4.0 |
| Glyceryl Monostearate and Diglycerides | 4.0 |
| Tween 80 | 4.2 |
| Purified Water | 58.0 |

### Comparative Example 2

A 2-isopropyl-5-(2'-phenylethyl)-1,3-benzenediol comparative ointment containing the commonly used photoprotective agent isooctyl salicylate was prepared according to the following table.

**Table 7. Ointments containing 2-ethylhexyl salicylate**

| Ingredients | Dosage (%,w/w) |
|---|---|
| 2-isopropyl-5-(2'-phenylethyl)-1,3-benzenediol(wt%) | 0.3 |
| Light Liquid Paraffin(wt%) | 9 |
| Isopropyl Myristate(wt%) | 11.8 |
| Medium-chain Triglycerides(wt%) | 11.7 |
| Isooctyl Salicylate (OSAL)(wt%) | 5 |
| White Vaseline (wt%) | Qs to100 |

### Investigating the effect of antioxidants on photostability

Color changes of Examples 8-13 and comparative preparations under illumination

2-isopropyl-5-(2'-phenylethyl)-1,3-benzenediol is sensitive to light and is easily degraded by light to generate colored substances, resulting in skin pigmentation, and light exposure can also reduce the content of the active ingredient in the preparation, affecting the efficacy. Parallel light experiments were conducted on the ointments of Examples 8-13 of the present invention, a cream without an antioxidant (Comparative Example 1), and an ointment containing the commonly used light protection agent isooctyl salicylate (Comparative Example 2).

Take equal amounts of the above samples and spread them flat in glass transparent containers of the same size, place them in a light box with a light intensity of LUX5000+84µw/cm²/h, and take samples for observation on day 0, day 4 (simulating 4 hours of outdoor sunlight on a sunny summer day) and day 12(ICH light stability test conditions).

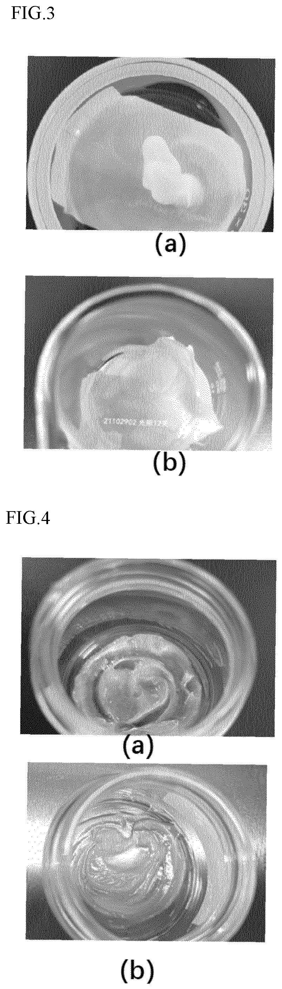

FIG. 3 (a) and (b) are photographs of the appearance changes of the cream preparation of Comparative Example 1 after 0 days and 12 days of illumination, respectively. FIG. 4 (a) and (b) are photographs of the appearance changes of Example 10 of the present invention after 0 days and 12 days of illumination, respectively. After being placed in a light box for 12 days, the color of the cream sample of Comparative Example 1 changed from milky white to dark yellow. The color of the ointment containing the light protection agent isooctyl salicylate (Comparative Example 2) also changed from light yellow to light brown, a significant change (illustration omitted). The degree of color change of Examples 8-13 containing antioxidants was significantly lower than that of Comparative Examples 1 and 2, with no obvious color change (illustration omitted). The ointment preparation of Example 10 performed best, with almost no color change, as shown in FIG. 4.

It can be seen that the light stability of the ointment of the present invention is significantly better than that of a cream without an antioxidant and an ointment containing the common antioxidant isooctyl salicylate. The present invention greatly improves the light resistance of the preparation, greatly reduces the color darkening problem and content reduction problem of the skin-surface preparation caused by light, and solves a major clinical limitation that similar preparations are prohibited from being used on the head and face in the instructions.

### Effects of different types and contents of antioxidants on active substances and related substances

Equal amounts of samples of Examples 8-12 containing antioxidants of different types and contents, Example 13 without an antioxidant, and Comparative Example 2 containing the light protection agent isooctyl salicylate were taken and spread in glass light-transmitting containers of the same size. They were placed in a light box with a light intensity of LUX5000+84µw/cm²/h. The content of active substances and related substances in the preparations were measured on day 0, day 4, and day 12. The results are shown in Tables 8 and 9, respectively.

**Table 8. Changes in the content of active substances**

| | Type of antioxidants | Content on day 0 (%) | Content on day 4 (%) | Content on day 12 (%) |
|---|---|---|---|---|
| Example 8 | 0.5% BHT | 100.0 | 97.5 | 69.2 |
| Example 9 | 0.5% VE | 98.8 | 97.5 | 17.0 |
| Example 10 | 0.1% PG | 99.4 | 98.6 | 98.3 |
| Example 11 | 0.5% BHT + 0.05% PG | 98.8 | 97.2 | 80.6 |
| Example 12 | 0.2% BHT + 0.1% PG | 101.4 | 105.8 | 100.2 |
| Example 13 | antioxidant-free ointment | 96.7 | 78.1 | 41.9 |
| Comparative Example 2 | 5% OSAL | 95.5 | 82.9 | 11.7 |

**Table 9. Related substances produced by different antioxidants**

| | Example 13 (antioxidant-free) | Example 10 (0.1% PG) | Example 11 (0.5% BHT + 0.05% PG) |
|---|---|---|---|
| Relative retention time of impurities | Content of related substance after 12 days of light exposure (%) | | |
| 0.18 | 0.08 | ND | ND |
| 0.25 | 0.19 | ND | ND |
| 0.32 | 0.21 | ND | ND |
| 0.37 | 0.11 | ND | ND |
| 0.47 | 0.05 | ND | ND |
| 0.52 | 0.08 | ND | ND |
| 0.60 | 0.11 | ND | ND |
| 0.70 | 0.09 | ND | ND |
| 0.82 | 0.06 | ND | ND |
| 0.84 | 0.31 | ND | ND |
| 0.89 | 6.28 | 0.20 | 0.18 |
| 1.19 | 0.10 | 0.09 | - |
| 1.22 | - | 0.82 | - |
| 1.27 | - | ND | 0.72 |
| 1.28 | - | ND | 0.10 |
| 1.31 | ND | - | ND |
| 1.45 | 0.76 | 0.26 | 0.19 |
| 1.49 | 0.07 | ND | ND |
| 1.52 | - | ND | 0.16 |
| 1.56 | ND | 0.06 | 0.13 |
| 1.63 | ND | ND | 0.06 |
| 1.67 | 0.22 | ND | 0.06 |
| 1.90 | - | ND | 0.08 |
| 1.95 | 0.33 | - | 0.09 |
| 2.12 | ND | ND | 1.02 |
| 2.35 | ND | ND | 1.30 |
| 2.63 | ND | ND | 0.11 |
| 2.74 | ND | ND | 0.2 |
| Total impurities | 10.20 | 1.79 | 4.32 |

| | | | |
|---|---|---|---|
| *Only report related substance with content of 0.05% or more, "-": related substance content less than 0.05%, "ND": not detected | | | |

As shown in Tables 8 and 9, the content of active substances in Example 13 without antioxidants decreased significantly when exposed to light, and the content of active substances in Example 10 containing propyl gallate remained unchanged after 12 days of light exposure, while the content of active substances in other antioxidant formulation decreased to a certain extent, among which the formulation containing dibutyl hydroxy toluene and vitamin E was less degraded after 4 days of light exposure, but was significantly degraded after 12 days of light exposure. The spectrum of light-degradation impurities of Example 13 without antioxidants is complex, and the active substances are degraded twice or even multiple times. In addition to the main degradation impurities with a relative retention time of 0.89, there are many chromatographic peaks with low content and impurities without ultraviolet absorption. After adding propyl gallate to the formulation, the degradation pathway of the active substances is changed, greatly reducing secondary degradation. The light stability of Example 11 containing 0.5% dibutyl hydroxy toluene and 0.05% propyl gallate is better than that of Example 8 containing only 0.5% dibutyl hydroxy toluene, but the light protection effect is weaker than that of Example 10 using 0.1% propyl gallate.

### Examples 14-15. 2-isopropyl-5-(2'-phenylethyl)-1,3-benzenediol ointment of different specifications

Isopropyl myristate was replaced by isopropyl palmitate, and 2-isopropyl-5-(2'-phenylethyl)-1,3-benzenediol ointment of different specifications was prepared according to the formulation in Table 10.

**Table 10. Different specifications of 2-isopropyl-5-(2'-phenylethyl)-1,3-benzenediol ointment**

| | Example 14 | Example 15 | Comparative Example 4 |
|---|---|---|---|
| 2-isopropyl-5-(2'-phenylethyl)-1,3-benzenediol(wt%) | 1 | 3 | 3 |
| White Vaseline(wt%) | 66.45 | 64.45 | 77.95 |
| Light Liquid Paraffin(wt%) | 16.5 | 9 | 9 |
| Isopropyl Palmitate(wt%) | 8 | 11.8 | 5 |
| Medium-chain Triglycerides(wt%) | 8 | 11.7 | 5 |
| Propyl Gallate(PG)(wt%) | 0.05 | 0.05 | 0.05 |

### Light stability of ointments of different specifications

The ointments of Examples 14 and 15 were subjected to a light stability test according to the aforementioned method. The results showed that after the 12 days of light exposure, the color of the ointments of Examples 14 and 15 hardly changed, and the content of the active substance remained unchanged. The antioxidants selected by the present invention can effectively protect 2-isopropyl-5-(2'-phenylethyl)-1,3-benzenediol ointments of different specifications.

### Physical stability of ointments of different specifications

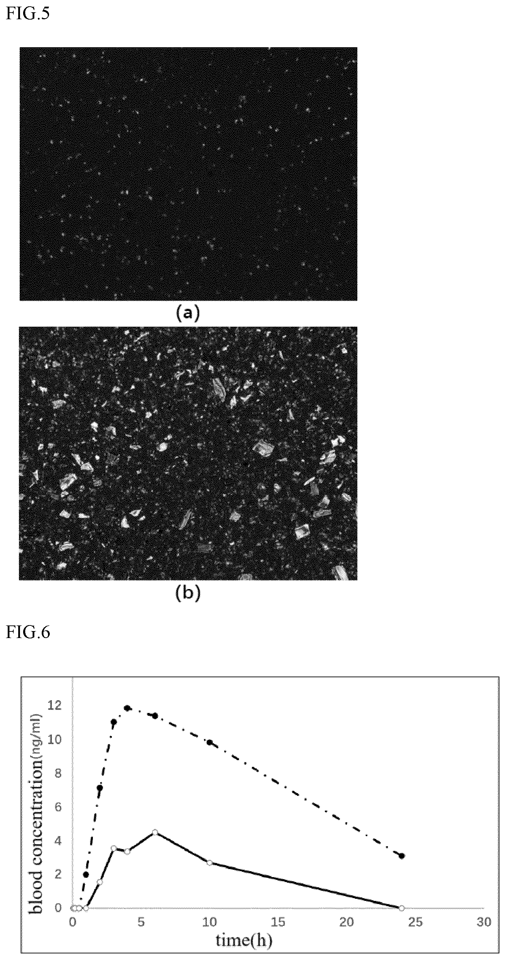

The ointment of Example 15 and Comparative Example 4 was stored at 5°C. After 3 months, the microstructure of the ointment and the dissolution of the active ingredient were observed by polarizing microscope. The results are shown in FIG. 5. Example 15 (FIG. 5a) is a formulation prepared according to the proportions of 2-isopropyl-5-(2'-phenylethyl)-1,3-benzenediol, isopropyl palmitate, and medium-chain triglycerides specified by the invention, no API crystals were observed in the preparation under polarizing microscope. After 3 months of storage at 5°C, 2-isopropyl-5-(2'-phenylethyl)-1,3-benzenediol can still be completely dissolved in the ointment matrix in the form of molecules without precipitation of crystals (the bright spot in FIG. 5a is the crystal phase of white vaseline, not the crystal of the drug). In contrast, the amount of isopropyl palmitate and medium-chain triglycerides in Comparative Example 4 (FIG. 5b) does not meet the ratio of the present invention (when preparing a 3% cream, the total amount of isopropyl palmitate (isopropyl myristate) and medium-chain triglycerides is 13-25%), and a large number of short rod-shaped drug crystals can be seen in the obtained preparation under a polarizing microscope, indicating that 2-isopropyl-5-(2'-phenylethyl)-1,3-benzenediol has crystallized and precipitated, and a solution-type ointment cannot be successfully obtained. This result further illustrates that the ratio range of isopropyl palmitate (and/or isopropyl myristate) to medium-chain triglycerides optimized by the present invention can prepare a stable solution-type ointment. The present invention provides a new method for optimizing the formulation ratio of preparations of different specifications, which greatly saves the time for formulation development and stability research.

### Examples 16-17

2-isopropyl-5-(2'-phenylethyl)-1,3-benzenediol ointment was prepared according to the formulation shown in Table 11. The in vivo exposure of preparations of different specifications was investigated using the SD rat model. The day before application, the hair on both sides of the spine of the animal's back was removed with electric clippers, and the cellophane was placed in the groove of the blank plaster patch and covered on the depilated area on the animal's back, fixed with medical tape, and the skin application area was 4×4cm² (application amount: 1.6g/kg).

**Table 11**

| | Example 16 | Example 17 |
|---|---|---|
| 2-isopropyl-5-(2'-phenylethyl)-1,3-benzenediol(wt%) | 0.3 | 1 |
| White Vaseline (wt%) | 67.50 | 67.50 |
| Light Liquid Paraffin(wt%) | 21.60 | 20.9 |
| Isopropyl Myristate(wt%) | 3.00 | 3.00 |
| Medium-chain Triglycerides(wt%) | 7.50 | 7.50 |
| Dibutyl hydroxy Toluene (wt%) | 0.1 | 0.1 |

As shown in FIG. 6, Example 16 and Example 17 were applied to the skin of rats, 2 hours after administration active ingredient could be detected in the blood. The 24-hour AUC of the preparations of 0.3% specification and 1% specification were 48 ng/ml*h and 182 ng/ml*h, respectively. This shows that the preparation has good transdermal performance, and the drug can be continuously and slowly released from the ointment matrix and penetrate the skin, and the blood drug concentration required for treatment is still maintained after 24 hours.

The protection content of the present invention is not limited to the above embodiments. Without departing from the spirit and scope of the inventive concept, changes and substitutions that can be thought of by those skilled in the art are included in the present invention, and the protection scope is defined by the attached claims.

## Claims

1. A solution-type ointments of a hydroxydiphenylethane compound, comprising 0.001-3% by weight of an active ingredient hydroxydiphenylethane compound, dissolved in the ointment matrix, and the remaining amount of the ointment matrix,
the ointment matrix comprises a hydrocarbon compound, a medium-chain triglyceride, and isopropyl palmitate and/or isopropyl myristate, wherein the weight ratio of the medium-chain triglyceride to the isopropyl palmitate and/or the isopropyl myristate is 1:4-3:1,
the hydrocarbon compound is selected from vaseline, liquid paraffin, or a mixture thereof,
the total amount of the medium-chain triglycerides and isopropyl palmitate and/or isopropyl myristate is 5-25% by weight.

2. The solution-type ointment according to claim 1, wherein when the specification of the ointment preparation is not higher than 1% by weight, the total amount of the medium-chain triglycerides and isopropyl palmitate and/or isopropyl myristate is 5-17% by weight, when the specification of the ointment preparation is 1-3% by weight, the total amount is 13%-25% by weight.

3. The solution-type ointment according to claim 1, wherein the hydroxydiphenylethane compound is selected from 1,2-bis(3,5-dihydroxy-4-isopropylphenyl)ethane, 2-isopropyl-5-(2'-phenylethyl)-1,3-benzenediol, 1-(3,5-dihydroxy-4-ethylphenyl)-2-phenylethane, 1,2-bis(3,5-dihydroxy-4-ethylphenyl)ethane, 1-(3,5-dihydroxy-4-butylphenyl)-2-phenylethane, 1,2-bis(3,5-dihydroxy-4-butylphenyl)ethane, preferably 2-isopropyl-5-(2'-phenylethyl)-1,3-benzenediol.

4. The solution-type ointment according to claim 1, wherein further comprises an antioxidant.

5. The solution-type ointment according to claim 1, wherein the antioxidant is selected from one or more of propyl gallate, butylated hydroxyanisole, and dibutyl hydroxytoluene.

6. The solution-type ointment according to claim 1, wherein the content of isopropyl palmitate and/or isopropyl myristate is 0.5-15% by weight, preferably 2-15% by weight.

7. The solution-type ointment according to claim 1, wherein the weight percentage of the active ingredient does not exceed 1.2×(0.445829×A+0.032300×B-0.000599×C+0.008112×A×B-0.003841×A×C)%, wherein A, B, and C represent the content of medium-chain triglycerides (%), the content of isopropyl myristate and/or isopropyl palmitate (%), and the content of hydrocarbon compounds (%), respectively, and A+B+C=100,
preferably, the weight percentage of the active ingredient does not exceed 0.9×(0.445829×A+0.032300×B-0.000599×C+0.008112×A×B-0.003841×A×C)%.

8. The solution-type ointment according to claim 1, wherein the active ingredient is present in a non-crystalline form in the ointment.

9. The solution-type ointment according to claim 1, wherein the content of the hydroxydiphenylethane compound is 0.005% by weight to 2% by weight, the content of the antioxidant is 0.01% by weight to 1% by weight, and the remainder is the ointment matrix.

10. The solution-type ointment according to claim 1, wherein the ointment does not contain water or contains less than 2% by weight of water.

11. The solution-type ointment according to any one of claims 1 to 10, wherein for use on the skin of the whole body, especially on the head and/or face.

12. A method for preparing the solution-type ointments of hydroxydiphenylethane compound according to any one of claims 1 to 10, wherein the active ingredient hydroxydiphenylethane compound and the antioxidant are added into a mixed solution of medium-chain triglycerides, isopropyl myristate and/or isopropyl myristate, heated and stirred to dissolve, and then added into a hydrocarbon compound melt by heating in advance, and stirred until uniform and then cooled to room temperature.
